(19) Europäisches Patentamt
European Patent Office
Office européen des brevets

(11) Publication number: 0 272 662 A2

# (12) EUROPEAN PATENT APPLICATION

(21) Application number: 87118919.7

(22) Date of filing: 21.12.87

(51) Int. Cl.[4]: C07C 11/18 , C07C 1/253

(30) Priority: 24.12.86 US 946071

(43) Date of publication of application:
29.06.88 Bulletin 88/26

(84) Designated Contracting States:
AT BE CH DE ES FR GB GR IT LI LU NL SE

(71) Applicant: UNION CARBIDE CORPORATION
39 Old Ridgebury Road
Danbury Connecticut 06817(US)

(72) Inventor: McCain, James Herndon
1987 Parkwood Road
Charleston West Virginia 25314(US)
Inventor: Kaiser, Steven William
900 Glendale Avenue
South Charleston West Virginia 25303(US)
Inventor: O'Connor, George Lawrence
844 Mathews Avenue
Charleston West Virginia 25302(US)

(74) Representative: Eggert, Hans-Gunther, Dr.
Räderscheidtstrasse 1
D-5000 Köln 41(DE)

## (54) Process for the production of dienes.

(57) Aldehydes can be converted to dienes by contacting the aldehydes with a zeolite or a silica molecular sieve. The zeolite or silica molecular sieves can achieve good conversion rates and selectivities for the reaction. The reaction is especially useful for the conversion of 2-methylbutyraldehyde to isoprene.

EP 0 272 662 A2

# PROCESS FOR THE PRODUCTION OF DIENES

## Field of the Invention

This invention relates to a process for the production of dienes. More specifically, this invention relates to a process for converting aldehydes to dienes by contacting the aldehydes with zeolites or silica molecular sieves. The process of the invention is especially useful for the production of isoprene from 2-methylbutyraldehyde.

## Background of the Invention

Production of isoprene in the United States of America amounts to approximately 200 million pounds per year, and world production amounts to approximately 2 billion pounds per year. Isoprene is used primarily in making cis-1,4-polyisoprene, a synthetic substitute for natural rubber. Polyisoprene and natural rubber are prime components of radial tires, whereas bias tires use other rubbers. Since radials are replacing bias tires, the demand for polyisoprene/natural rubber is increasing. Depending on the supply of natural rubber, more polyisoprene will be needed in the future.

At present, in the United States, all commercial production of isoprene is by extraction from $C_{5+}$ streams of olefin plants. The availability of isoprene from this source, however, depends on the output of ethylene and on the feedstock fed to the olefin plants. Heavier feedstocks such as naphtha produce more isoprene while lighter feedstocks such as liquified petroleum gas produce less. As the cracking stocks of olefin plants have become lighter in the recent past, the amount of isoprene produced has declined.

In Eastern Block countries, where ethylene is not produced in such large amounts as in the United States, isoprene is made in dedicated facilities. The Soviet Union is the premier isoprene manufacturer with over 1 billion pounds of capacity. The main synthetic route used is the Prins reaction of isobutene with formaldehyde followed by dehydration of the intermediate 4,4-dimethylmetadioxane (4,4-DMD) adduct.

Other processes available for the production of isoprene include, for example, the reaction of acetylene and acetone, followed by hydrogenation, to give dimethylvinylcarbinol, which is then dehydrated to yield isoprene.

A new and potentially very economical process for the production of isoprene is the dehydration of 2-methylbutyraldehyde (2-MBA). 2-MBA can be produced by hydroformylation of butene and so is an inexpensive raw material which can be manufactured in the large quantities needed for large scale commercial production of isoprene. The dehydration of 2-MBA to isoprene is catalyzed by acids and can be effected with reasonable selectivity. However, since isoprene itself is polymerized by strong acids such as sulfuric acid, such strong acids are not useful as isoprene catalysts.

A wide variety of catalysts, including both molecular sieves and various other materials, have been proposed for use in the aforementioned reactions for the production of isoprene and in chemically-similar reactions. For example, British Patent No. 673,547 describes a process for the conversion of 1-methoxy-3-methylbut-4-ene to isoprene using a silicate of aluminum as the catalyst.

U.S. Patent No. 3,253,051 to Yanagita et al. describes a process for the production of isoprene from a mixture of isobutylene and formaldehyde in which the catalyst employed is a mixture of an oxide or hydroxide of a metal such as iron with phosphoric acid, this mixture being supported on a suitable carrier, for example silica or alumina.

U.S. Patents No. 3,872,216 and 3,846,338, both to Kachalova et. al., describe methods for producing isoprene from a dioxane employing a tricalcium phosphate catalyst.

British Patent No. 1,385,348 describes the production of a diene from the corresponding aldehyde using an acidic dehydration catalyst, which can comprise inorganic acids, inorganic acid salts, acid anhydrides of an inorganic acid, or mixed anhydrides thereof either supported or unsupported. Specific catalysts mentioned include phosphoric acid, boric acid, silicic acid, titanic acid, boron phosphate, silicon borate, and silicon titanate.

British Patent No. 2,093,060 describes the dehydration of carbonyl compounds to dienes using as catalysts magnesium phosphate compositions, while British Patent No. 2,063,297 describes the use of alum and mixed alum sulfates as catalysts in the same reaction.

Zeolite molecular sieves have been used as catalysts for the production of isoprene by dehydration of starting materials other than 2-MBA. For example, Japanese Patent Application Publication No. 77/57104

describes the dehydration of 4,4-DMD in the gas phase over Group II metal-exchanged Zeolite X. Japanese Patent Application Publication No. 74/10924 describes the dehydration of 4,4-DMD or 4-methyl-5,6-dihydro-2H-pyran in the gas phase over a zeolite catalyst. Japan Patent Application Publication No. 77/36603 describes the dehydration of 4,4-DMD over a catalyst composed of Zeolite X and an acid clay. Nefedova et al., Neftekhimiya 19, 113 (1979) describe the dehydration of 2-methylbutane-2,3-diol using as catalysts Zeolites A, X, and Y. Ivanova and Kucherov, Neftekhimiya 10, 400 (1970) describe the use of Zeolite Y as a catalyst for the dehydration of dimethylvinylcarbinol.

A variety of catalysts have also been proposed for use in the dehydration of 2-methylbutyraldehyde to isoprene. U.S. Patent No. 1,033,180 describes the use of aluminum silicate as a catalyst. European Patent Application Publication No. 80,449 describes the use of acidic, heterogeneous catalysts, specifically boron phosphate and silica in this reaction. U.S. Patent No. 4,524,233 describes the use of boron phosphate admixed with graphite and treated with amines and steam as a 2-MBA to isoprene catalyst. U.S.S.R. Patent No. 721,116 and Bol'shakov et al., Neftekhimiya 23, 183 (1983) describe the use of boron phosphate as a catalyst for this reaction. Irodov, Smirnov and Kryukov, Zh. Org. Khim, 18, 1401 (1982) describe the use of amorphous aluminum, boron and calcium phosphates as catalysts for the conversion of 2-MBA to isoprene.

U.S. Patent No. 4,560,822 to Hoelderich et al. (which issued December 24, 1985 on Application Serial No. 730,687 filed May 3, 1985 and claiming priority of West German Patent Application No. 34 19 379 filed May 24,1984) describes and claims a process for the dehydration of aldehydes to dienes (including the dehydration of 2-methylbutyraldehyde to isoprene) at elevated temperatures using a zeolite as a catalyst.

It has now been discovered that the dehydration of 2-methylbutyraldehyde to isoprene can be efficiently conducted using zeolites or silica molecular sieves as catalysts. The same catalysts can be used for the dehydration of other aldehydes to the corresponding dienes.

## Summary of the Invention

This invention provides a process for dehydrating an aldehyde to a diene, which process comprises contacting the aldehyde with a zeolite or a silica molecular sieve under conditions effective to convert the aldehyde to the diene.

The aldehyde used in the process of the present invention may be, for example, an aldehyde of the formula

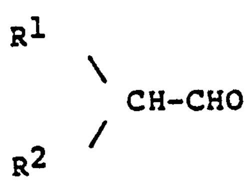

I

where $R^1$ and $R^2$ are identical or different, $R^1$ is alkyl with 1 to 3 carbon atoms or hydrogen and $R^2$ is alkyl with 1 to 8 carbon atoms or a hydrocarbon radical which together with $R^1$ forms a cyclohexyl radical, with the conversion being effected at from 150° to 600°C over a zeolite as a catalyst. The process of the invention may be used for the preparation of a diene of the formula

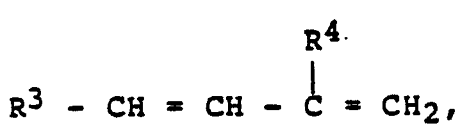

II

where $R^3$ is methyl, ethyl or hydrogen and $R^4$ is methyl or ethyl, by passing 2-methylbutanal, 2-methylpentanal, 2-ethylhexanal, 3-methylbutanal, isovaleraldehyde or pivalaldehyde at from 300° to 500°C over a zeolite as a catalyst. The zeolite is conveniently an aluminosilicate zeolite, such as one having a pentasil (ZSM-5 type) structure. Borosilicate and iron silicate zeolites may also be used.

The aldehyde used in the process of the present invention is desirably one containing from about 4 to about 6 carbon atoms. A specific preferred aldehyde for use in the process is 2-methylbutyraldehyde.

Detailed Description of the Invention

The zeolites and silica molecular sieves used in the process of the present invention are well-known to those skilled in the molecular sieve art. Representative zeolites include Y type zeolites, X type zeolites, zeolite beta (U.S. Patent No. 3,308,069), zeolite L, zeolite omega, zeolite ZSM-3 (U.S. Patent No. 3,415,736), ZSM-5 zeolite (pentasil structure) sold by Mobil Oil, or the ZSM-5 type zeolite known as LZ-105 produced by Union Carbide Corporation, other ZSM-type zeolites, mordenite, faujasite and mixtures thereof. Natural zeolites such as ferrierites, may also be used. Conveniently, the zeolite used in the process of the present invention is an X or Y type zeolite, or a Y type zeolite which has been subjected to removal of aluminum from the lattice framework. Y type zeolites include to those disclosed in U.S. Patent Nos.: 3,835,032; 3,830,725; 3,293,192; 3,449,070; 3,839,539; 3,867,310; 3,929,620; 3,929,621; 3,933,983; 4,058,484; 4,085,069; 4,175,059; 4,192,778; 3,676,368; 3,595,611; 3,594,331; 3,536,521; 3,293,192; 3,966,643; 3,9665,882; and 3,957,613. Specific Y type zeolites which have been demonstrated to be useful in the process of the present invention include those sold commercially by Union Carbide Corporation under the tradenames Y-52, Y-72 and Y-82. Y-82 Zeolite gives especially good results, particularly if treated to incorporate phosphorous before the zeolite is used in the process of the present invention; such phosphorous treatment of Y-82 zeolite can be achieved in the manner described in U.S. Patent 4,044,065. Preparation of the phosphorous-treated Y-82 zeolite is illustrated in Example 2 below.

Removal of aluminum from the lattice framework of a Y type zeolite may be achieved by the processes described in U.S. Patent No. 4,503,023 to Breck. These processes involve contacting a Y type zeolite with a fluorosilicate salt under controlled temperature and concentration conditions such that the fluorosilicate extracts aluminum from the lattice framework, and replaces this aluminum with silicon, without substantially affecting the crystallinity of the zeolite.

Removal of aluminum from the lattice framework of a zeolite may also be achieved by thermal processes involving prolonged exposure of the zeolite to steam, as described for example in Canadian Patent No. 1,131,195 and British Patent No. 2,014,970. It has been found that, when using in the process of the present invention a zeolite from which aluminum has been extracted by thermal treatment, the selectivity to the diene is improved if the aluminum-extracted zeolite is first washed with acid. Such acid washing may be effected by refluxing the aluminum-extracted zeolite with moderately concentrated hydrochloric acid for a period of a few hours, then rinsing the zeolite with dilute nitric acid until the washings are chloride-free.

Aluminum-extracted zeolites useful in the process of the present invention include LZ-10, LZ-20 and LZ-210 zeolites, with LZ-10 and LZ-20 zeolites giving particularly good results.

The catalysts used in the process of the present invention may be modified by depositing or impregnating them with cations, anions or salts so as to improve its efficacy as a catalyst in the process of the present invention. Techniques which may be employed to effect the deposition or impregnation of a zeolite or silica molecular sieve are generally known in the art. Such procedures may involve such procedures as (1) impregnating the catalyst with a solution comprising a solvent or solubilizing agent of one or more such modifying materials in an amount sufficient to deposit the desired weight of such materials in the catalyst and/or (2) exchanging the catalyst with a solution containing the modifying material. The impregnation or deposition of the modifying materials may generally be accomplished by heating the catalyst at an elevated temperature to evaporate any liquid present to effect deposition or impregnation of the modifying material on to the interior and/or exterior surface of the catalyst, or by the exchange of cations present in the catalyst with cations that provide for the desired properties. Alternatively, the modifying material may be formed on the catalyst from an emulsion or slurry containing the modifying material by heating the catalyst. Impregnation or exchange procedures are generally the preferred techniques because they utilize and introduce the modifying material more efficiently than other procedures such as coating procedures since a coating procedure is generally not able to effect substantial introduction of the modifying material on to the interior surfaces of the catalyst. In addition, coated materials are more generally susceptible to the loss of the modifying materials by abrasion.

Suitable modifying materials include alkali metals, alkaline earth metals, transition metals and the salts thereof including inorganic and organic salts such as nitrates, halides, hydroxides, sulfates and carboxylates. Other modifying materials generally employed in the art are also believed to be employable in the catalysts used in the process of the present invention.

More specifically, the catalytic activity of zeolites, especially LZ-10 and LZ-20, in the process of the present invention, may be improved by incorporating therein any one or more of an alkali metal, an alkaline earth metal, a rare earth metal, zinc, cadmium, manganese, aluminum, bismuth and phosphorus. Preferred metals for this purpose are magnesium, calcium, barium, strontium and lanthanum. Advantageously, an

aluminum-extracted zeolite is acid washed before being loaded with such metals. Specific preferred metal-loaded zeolites for use in the process of the present invention are acid-washed, calcium-treated LZ-10, acid-washed, magnesium-treated LZ-10, acid-washed, barium-treated LZ-10, acid-washed, strontium-treated LZ-10, acid-washed, lanthanum-treated LZ-10, phosphorous-treated LZ-10 and acid-washed, magnesium-treated LZ-20. Appropriate techniques for introducing the preferred metals into LZ-10 and LZ-20 zeolites are illustrated in the examples below.

It should be noted that acid washing and metal incorporation of certain zeolites, and in particular the preferred LZ-10 and LZ-20 aluminum-extracted zeolites, may in some cases at least partially degrade the microporous nature of the zeolite molecular sieve, rendering the acid-washed, metal-loaded material partially amorphous rather than fully crystalline. Although such degraded materials do not appear to be, strictly speaking, molecular sieves, they are usable in the process of the present invention, and the term "zeolite" is used herein to include such degraded materials.

Among the specific silicate molecular sieves which have been found to give acceptable results in the process of the present invention are silicalite (also known, and hereinafter sometimes referred to, as S-115) and S-130. The silicate molecular sieves may also metal-loaded in a manner similar to that already described for zeolites.

As already mentioned, in the process of the present invention an aldehyde is contacted with a zeolite or a silica molecular sieve under conditions effective to convert the aldehyde to the corresponding diene. As is well known to those familiar with the use of molecular sieves as catalysts, the reaction rate and product distribution of a molecular sieve catalyzed reaction is strongly influenced by the pore size of the molecular sieve, since the catalytic activity of molecular sieves appears to depend upon the ability of the reactants and products to diffuse through the pores of the molecular sieve. The pore sizes of molecular sieves vary over a wide range and, since dehydration of aldehydes such as 2-methylbutyraldehyde to the corresponding dienes such as isoprene involves the diffusion of molecules of moderate size through the non-zeolitic molecular sieve, the catalyst used in the process of the invention should be selected to provide a sufficiently large pore size to accommodate these molecules.

As already noted, the dehydration of 2-MBA to isoprene is catalyzed by acids, but isoprene itself is polymerized by strong acids, as are other dienes. Consequently, the presence of some acid sites in the molecular sieve catalyst used in the process of the present invention is desirable, but very strongly acid sites should be avoided.

The particle size of the molecular sieve catalyst also appears to influence its activity, and it is preferred that the molecular sieve be used in finely divided form.

The process of the present invention may, at least in theory, be conducted with the aldehyde in the liquid phase. However, it is preferred that the process of the present invention be operated as a heterogeneous, gas phase reaction with the aldehyde in the gaseous phase.

In such a gas phase process, the aldehyde may be mixed with an inert carrier gas while being contacted with a non-zeolitic molecular sieve, although the use of such an inert carrier gas is not essential in the process of the present invention, which can be operated using pure aldehyde as the gaseous feed. The degree of dilution of the aldehyde with such an inert carrier gas may vary considerably depending upon the conditions used and any process constraints restricting the use of inert diluents. (For example, in commercial production, the use of very large quantities of inert carrier gas is disadvantageous due to the cost of pumping large volumes of gas and increased difficulty in isolating the product, which increase the energy costs of the process.) A variety of inert carrier gases may be used; nitrogen, carbon dioxide and hydrocarbons are all satisfactory inert carrier gases in the process of the present invention. Steam may also be used and the presence of steam in the reactor may tend to reduce coke formation in the catalyst and hence may increase the period before regeneration of the catalyst (discussed in more detail below) is required.

Selection of the temperature and pressure at which the process of the present invention is to be conducted typically involves a compromise between selectivity to the diene and conversion of the aldehyde. It is recommended that the process of the present invention be conducted at a temperature in the range of about 150°C to about 500°C; below this temperature range, the reaciton tends to proceed quite slowly, while at very high temperatures, the selectivity to isoprene (or other diene) tends to decrease. The preferred temperature range is from about 250°C to about 400°C. For obvious reasons, it is desirable to adjust the temperature and the other reaction parameters discussed below so as to ensure that the reaction is run at a conversion of at least about 10%, and preferably at least about 20%, and at a selectivity to the diene of at least about 50%, and preferably at least about 70%. It should be noted that the conversion and selectivity achieved with a given material may vary considerably with its exact composition and the procedure and raw materials used to prepare a given specimen of the material.

The reaction may be run over a wide range of pressures, for example from 0.1 to 200 atmospheres (approximately 0.01 to 10 MPa.) or more, but for practical reasons it is recommended that the process of the present invention be carried out at a pressure of from 0.1 to 50 atmospheres (approximately 0.01 to 5.1 MPa). It has been found that satisfactory conversions and selectivities can be achieved by running the process of the invention under atmospheric pressure.

The process of the present invention can be carried out over a wide range of weight hourly space velocities of the aldehyde. For example, in the case of 2-MBA, weight hourly space velocities of from 0.01 to 40 may be employed, although preferably the weight hourly space velocity is in the range of from 1 to 10.

The selectivity and conversion achieved in the process of the present invention may vary in rather a complex manner with the time for which the catalyst has been used in the process. The catalysts can gradually become deactivated; however, the deactivated catalyst can readily be regenerated by heating in air at an appropriate temperature (typically about 500°C) and for an appropriate period (typically one hour).

In carrying out the process of the present invention, the zeolite or silica molecular sieves may be admixed (blended) or provided sequentially to other materials which may provide some property which is beneficial under process conditions, such as improved temperature resistance or improved catalyst life by] minimization of coking, or which are simply inert under the process conditions used. Such materials may include synthetic or naturally-occurring substances as well as inorganic materials such as clays, silicas, graphite, aluminas, crystalline aluminosilicate zeolites, metal oxides and mixtures thereof. In addition, the zeolite or silica molecular sieves may be formed with materials such as silica, alumina, silica-alumina, silica-magnesia, silica-zirconia, silica-thoria, silica-berylia, and silica-titania, as well as ternary compositions, such as silica-alumina-thoria, silica-alumina-zirconia and clays present as binders. The relative proportions of the above materials and the zeolite or silica molecular sieves may vary widely.

The process of the present invention may be carried out using, for example, a fixed bed reactor, a fluid bed reactor, or a slurry reactor. The optimum size and shape of the catalyst particle will depend on the type of reactor used. In general, for fluid bed reactors, a small, spherical catalyst particle is preferred for easy fluidization. With fixed bed reactors, larger catalyst particles are preferred so the back pressure within the reactor is kept reasonably low.

The following Examples are provided to further illustrate the process of the present invention, but are not limitative thereof.

## EXAMPLES

### Experimental Conditions

Two different reactors were used in the experiments, namely a sparged system reactor and a pumped system reactor. In both reactors, approximately 0.50 grams of the catalyst under test was mixed with approximately 2.50 grams of 20-40 U.S. mesh quartz chips, and the resultant mixture packed into a reactor consisting of a 3/8 inch (9mm.) diameter by 3 inch (76mm.) stainless steel tube.

In the sparged system, four such reactors were mounted in parallel in a thermostated, fluidized sand bath. Each reactor was fed a gaseous nitrogen/2-methylbutyraldehyde mixture containing 5% by volume of 2-MBA, this mixture being prepared by sparging nitrogen through liquid 2-MBA at room temperature. The total flow rate of the nitrogen/2-MBA mixture through each reactor was 4ml./minute. Successive runs were conducted at 275, 350 and 425°C.

The products of the reaction were passed to a stream selector valve, which routed each reactor effluent in succession to the inlet valve of a gas chromatograph. The conversions and selectivities reported using this method are approximate. However, the method was sufficiently accurate to allow an approximate comparison of the various catalysts for the conversion of 2-MBA to isoprene, and thus provided a rough screening to identify the specific catalysts worthy of more detailed investigation.

In the pumped system, only one reactor was placed in the fluidized sand bath. The reactor was connected to a preheater, to which was fed about 1.5ml./hour of liquid 2-MBA and 1.0ml./minute of nitrogen gas, so that the gaseous mixture flowing from the preheater into the reactor at a rate of approximately 4ml./minute consisted of approximately 75% by volume of 2-MBA and 25% by volume of nitrogen. The temperature of the reactor was usually adjusted to give a conversion of about 30%.

The reaction products were collected downstream from the reactor in a dry ice cold trap. The material collected in the cold trap was analyzed by gas chromatography. Several samples were taken from each

reaction and the results given below are averages.

All conversions and selectivities given hereinafter are percentage conversions and selectivities for the conversion of 2-MBA to isoprene. Any methylisopropyl ketone produced is counted as unreacted 2-MBA since the ketone can be recycled.

## Example 1

This Example reports the results of screening tests carried out using the sparged system described above in order to find effective zeolite or silica molecular sieves for the conversion of 2-MBA to isoprene.

Various zeolite or silica molecular sieves were tested for their ability to catalyze the conversion of 2-MBA to isoprene using the sparged system described above. The results of these experiments are reported in Table 1 below. In this Table, the chemical symbol for a metal preceding the name of the molecular sieve indicates that the molecular sieve was loaded with that element, while "aw" following the name of the molecular sieve indicates that the molecular sieve was acid-washed. The techniques used for the acid washing and loading of the molecular sieve with various elements are described in detail in other Examples below. LZ-10, LZ-20, LZ-210, S-130, S-115, LZ-134 and M-8 are all materials discovered and/or manufactured by Union Carbide Corporation.

TABLE I

| Molecular sieve | Conversion/Selectivity at: 275°C | 350°C | 425°C |
|---|---|---|---|
| Cs LZ-10 aw | 0/- | 3/4 | 5/38 |
| K LZ-10 aw | 3/11 | 6/59 | 8/49 |
| Li LZ-10 aw | 4/31 | 9/51 | 34/58 |
| Ba LZ-10 aw | 3/100 | 15/100 | 49/96 |
| Sr LZ-10 aw | 3/100 | 18/100 | 57/87 |
| Ca LZ-10 aw | 8/100 | 27/100 | 58/78 |
| Mg LZ-10 aw | 16/100 | 58/91 | 88/75 |
| Mn LZ-10 aw | 5/58 | 20/73 | 43/71 |
| Zn LZ-10 aw | 14/67 | 32/71 | 54/51 |
| Cd LZ-10 aw | 13/48 | 32/65 | 48/61 |
| Al LZ-10 aw | 25/16 | 39/2 | 44/2 |
| Sb LZ-10 aw | 8/41 | 22/27 | 41/19 |
| Ba LZ-10 | 69/1 | 57/1 | 67/1 |
| Ca LZ-10 | 24/5 | 38/8 | 54/6 |
| Mg LZ-10 | 38/0 | 83/0 | 95/0 |
| Ba Y-82 | 33/6 | 66/38 | 82/16 |
| Ca Y-82 | 34/4 | 74/47 | 90/24 |
| Mg Y-82 | 75/0 | 86/0 | 96/7 |
| Ba S-115 | 26/3 | 55/3 | 84/2 |
| Ca S-115 | 33/3 | 69/2 | 90/0 |
| Mg S-115 | 57/0 | 61/0 | 90/0 |
| Ba LZ-105 | 25/53 | 62/2 | 80/3 |
| Ca LZ-105 | 32/31 | 64/2 | 86/1 |
| Mg LZ-105 | 27/3 | 51/3 | 80/1 |
| Ba LZ-210 | 15/39 | 34/58 | 80/57 |
| Ca LZ-210 | 28/4 | 33/64 | 45/47 |
| Mg LZ-210 | 58/0 | 41/58 | 86/51 |
| P Y-82 | 8/6 | 15/77 | 31/89 |
| P S-115 | 14/89 | 27/90 | 18/69 |
| P LZ-105 | 28/28 | 50/79 | 28/47 |
| P LZ-10 | 25/57 | 51/54 | 65/35 |
| LZ-10 aw | 6/100 | 15/82 | 51/51 |
| LZ-10 | 43/1 | 76/1 | 91/1 |
| Y-82 | 19/2 | 65/1 | 89/1 |
| Y-82 (pre-tested) | 11/69 | 36/77 | 45/39 |
| S-115 | 21/37 | 35/6 | 50/3 |
| S-130 | 36/3 | 78/2 | 91/2 |
| LZ-210 | 28/3 | 80/1 | 91/44 |
| LZ-105 | 19/10 | 58/3 | 76/2 |
| M-8 (mordenite) | 16/70 | 44/72 | 70/55 |
| LZ-134 | 14/76 | 21/75 | 36/58 |
| Norton Zeolon 700 (ferrierite) | 7/70 | 30/80 | 53/70 |

Example 2

Samples of somer of the more promising catalysts tested in Example 1 above were further tested in the pumped system described above. The results are shown in Table II below.

TABLE II

| Molecular Sieve | Temp. °C | Conversion | Selectivity |
|---|---|---|---|
| Ba LZ-10 aw | 415 | 14 | 66 |
| Sr LZ-10 aw | 415 | 10 | 65 |
| Ca LZ-10 aw | 415 | 19 | 73 |
| Mg LZ-10 aw | 341 | 11 | 79 |
| Mg LZ-10 aw | 415 | 42 | 68 |
| P Y-82 | 415 | 12 | 81 |
| Y-82 | 379 | 30 | 70 |
| M-8 | 365 | 24 | 74 |

Example 3

This example shows the good selectivity obtained using phosphorus-treated Y-82 as catalyst.

Phosphorus-treated Y-82 zeolite was prepared by the process described in Example 1 of U.S. Patent 4,044,065. 5.0 Grams of Y-82 zeolite and 2.0 ml. of trimethylphosphite were refluxed for 72 hours in 25 ml. of octane solvent. The solid product was separated by filtration, washed successively with pentane, methylene chloride and pentane, and dried at 110°C and then at 500°C for three hours.

The resultant phosphorus-treated Y-82 was tested for its catalytic ability using the pumped system described above, with the sand bath at the temperatures specified in Table III below, using a 2-MBA flow rate of 1.5ml. per hour of liquid and a nitrogen flow rate of 1ml. per minute; the weight hourly space velocity of the 2-MBA feed was 3. The results are given in Table III below, which illustrates the variation in catalytic activity with operating time of the catalyst.

Table III

| Time, hours | Temp. °C | Conversion | Selectivity |
|---|---|---|---|
| 0.7 | 309 | 2 | 63 |
| 1.4 | 316 | 4 | 83 |
| 2.1 | 365 | 7 | 80 |
| 3.0 | 365 | 10 | 85 |
| 3.7 | 413 | 14 | 81 |
| 4.5 | 413 | 11 | 78 |

Example 4

This example shows the results obtained in the sparged system described above using phosphorus-treated S-115 silica molecular sieve as catalyst.

Phosphorus treatment of S-115 was effected in the same manner as in Example 3, and the resultant phosphorus-treated S-115 tested in the sparged system described above. The results obtained are shown in Table IV below.

Table IV

| Time, hours | Temp. °C | Conversion | Selectivity |
|---|---|---|---|
| 1 | 275 | 14 | 89 |
| 6 | 350 | 27 | 90 |
| 9 | 425 | 18 | 69 |

Example 5

This example shows the results obtained in the sparged system described above using phosphorus-treated LZ-10 zeolite as catalyst.

Phosphorus treatment of LZ-10 was effected in the same manner as in Example 3, and the resultant phosphorus-treated LZ-10 tested in the sparged system described above. The results obtained are shown in Table V below.

Table V

| Time, hours | Temp. °C | Conversion | Selectivity |
|---|---|---|---|
| 2 | 275 | 25 | 57 |
| 6 | 350 | 51 | 54 |
| 9 | 425 | 65 | 35 |

Example 6

Y-82 zeolite was used as a catalyst in the pumped system described above. The results obtained are shown in Table VI below.

Table VI

| Time, hours | Temp. °C | Conversion | Selectivity |
|---|---|---|---|
| 0.7 | 314 | 15 | 36 |
| 1.5 | 315 | 19 | 38 |
| 2.2 | 359 | 45 | 37 |
| 3.0 | 378 | 35 | 67 |
| 3.7 | 379 | 33 | 69 |
| 4.5 | 379 | 29 | 70 |
| 5.2 | 379 | 27 | 71 |
| 6.0 | 379 | 24 | 71 |

Example 7 (Control)

This example shows the low conversion and selectivity obtained using only quartz chips in the pumped reactor.

Example 3 was repeated except that only 2.50 grams of quartz chips, without any non-zeolitic molecular sieve, were charged to the reactor. The results obtained at 316°C are shown in Table VII below.

Table VII

| Time, hours | Conversion | Selectivity |
|---|---|---|
| 1 | 1 | 0 |
| 2 | 1 | 0 |
| 3 | 1 | 0 |
| 4 | 1 | 0 |
| 5 | 1 | 0 |

Example 8 (Control)

This example shows the conversion and selectivity obtained using only quartz chips in the sparged reactor.

Example 1 was repeated except that only 2.50 grams of quartz chips, without any non-zeolitic molecular sieve, were charged to the reactor. The results obtained are shown in Table VII below.

Table VIII

| Time, hours | Temperature °C | Conversion | Selectivity |
|---|---|---|---|
| 4 | 275 | 8 | 31 |
| 8 | 350 | 23 | 31 |
| 11 | 425 | 34 | 11 |

Example 9

S-115 was used as a catalyst under the same conditions as in Example 3, except that the temperature of the sand bath was 310°C. The results obtained are shown in Table IX below.

Table IX

| Time, hours | Conversion | Selectivity |
|---|---|---|
| 1 | 14 | 22 |
| 2 | 5 | 13 |
| 3 | 3 | 29 |

Example 10

This example shows the results obtained in the pumped system using calcium-treated, acid-washed LZ-10 as catalyst.

50 Grams of LZ-10 was refluxed in 12 percent hydrochloric acid for three hours and the solids separated by filtration. The separated molecular sieve was then washed with dilute nitric acid (pH 3.0) until the washings were substantially free of chloride, as evidenced by little or no precipitate with silver chloride. A 5.0 gram portion of the acid-washed LZ-10 thus obtained was treated overnight at 90°C with 45 ml. of 10 percent aqueous calcium acetate solution, and this overnight treatment repeated twice with fresh batches of the calcium acetate solution. The calcium-treated molecular sieve produced by this treatment was then treated overnight at 90°C with distilled water, and this overnight treatment repeated once with a fresh batch of distilled water. Finally, the treated molecular sieve was dried in an oven at 200°C and then heated for 2 hours at 500°C.

The calcium-treated, acid-washed LZ-10 was used as a catalyst in the pumped reactor system described above, and the results obtained are shown in Table X below.

**Table X**

| Time, hours | Temp. °C | Conversion | Selectivity |
|---|---|---|---|
| 0.7 | 210 | 1 | 50 |
| 1.5 | 278 | 1 | 48 |
| 2.2 | 278 | 1 | 56 |
| 3.0 | 313 | 4 | 78 |
| 3.7 | 342 | 4 | 80 |
| 4.5 | 343 | 21 | 69 |
| 5.5 | 415 | 20 | 71 |
| 6.2 | 415 | 18 | 69 |

Example 11

This example shows the results obtained in the pumped system using magnesium-treated, acid-washed LZ-10 as catalyst.

Magnesium-treated, acid-washed LZ-10 was prepared in the same way as in Example 10, but substituting magnesium acetate for the calcium acetate, and tested in the pumped system described above. The results obtained are shown in Table XI below.

**Table XI**

| Time, hours | Temp. °C | Conversion | Selectivity |
|---|---|---|---|
| 0.7 | 278 | 4 | 75 |
| 1.5 | 278 | 7 | 67 |
| 2.2 | 278 | 7 | 67 |
| 3.0 | 278 | 3 | 76 |
| 3.7 | 341 | 12 | 69 |
| 4.5 | 341 | 12 | 70 |
| 5.2 | 415 | 37 | 69 |
| 6.0 | 415 | 45 | 64 |

Example 12

This example shows the results obtained in the pumped system using barium-treated, acid-washed LZ-10 as catalyst.

Barium-treated, acid-washed LZ-10 was prepared in the same way as in Example 10, but substituting barium acetate for the calcium acetate, and tested in the pumped system described above. The results obtained are shown in Table XII below.

Table XII

| Time, hours | Temp. °C | Conversion | Selectivity |
|---|---|---|---|
| 0.7 | 278 | 1 | 76 |
| 1.5 | 344 | 2 | 74 |
| 2.2 | 342 | 2 | 80 |
| 3.0 | 357 | 12 | 69 |
| 3.7 | 415 | 17 | 65 |
| 4.5 | 416 | 16 | 61 |
| 5.2 | 415 | 14 | 62 |

Example 13

This example shows the results obtained in the pumped system using strontium-treated, acid-washed LZ-10 as catalyst.

Strontium-treated, acid-washed LZ-10 was prepared in the same way as in Example 10, but substituting strontium acetate for the calcium acetate, and tested in the pumped system described above. The results obtained are shown in Table XIII below.

Table XIII

| Time, hours | Temp. °C | Conversion | Selectivity |
|---|---|---|---|
| 1.0 | 278 | 1 | 11 |
| 1.7 | 278 | 5 | 18 |
| 2.5 | 278 | 2 | 76 |
| 3.2 | 342 | 2 | 76 |
| 3.7 | 343 | 10 | 71 |
| 4.5 | 343 | 12 | 64 |
| 5.3 | 414 | 11 | 64 |
| 6.0 | 414 | 10 | 62 |

Example 14

This example shows the results obtained in the pumped system using magnesium-treated, acid-washed LZ-20 as catalyst.

Magnesium-treated, acid-washed LZ-20 was prepared in the same way as in Example 10, but substituting LZ-20 for the LZ-10, and tested in the pumped system described above, using a bath temperature of 317°C. The results obtained are shown in Table XIV below.

Table XIV

| Time, hours | Conversion | Selectivity |
|---|---|---|
| 1 | 7 | 65 |
| 2 | 6 | 65 |
| 3 | 6 | 65 |
| 4 | 5 | 64 |

Example 15

This example shows the results obtained in the pumped system using acid-washed LZ-20 as catalyst.

Acid-washed LZ-20 was prepared using the acid washing step (but not the calcium treatment step) of Example 10 and substituting LZ-20 for the LZ-10. The acid-washed LZ-20 thus prepared was tested in the pumped system described above, using a bath temperature of 314°C. The results obtained are shown in Table XV below.

Table XV

| Time, hours | Conversion | Selectivity |
|---|---|---|
| 1 | 9 | 41 |
| 2 | 6 | 41 |

Example 16

This example shows the results obtained in the pumped system using acid-washed LZ-10 as catalyst.

Acid-washed LZ-10 was prepared using the acid washing step but not the calcium treatment step of Example 10. The acid-washed LZ-10 thus prepared was tested in the pumped system described above, and the results obtained are shown in Table XVI below.

Table XVI

| Time, hours | Temp. °C | Conversion | Selectivity |
|---|---|---|---|
| 1 | 307 | 4 | 54 |
| 2 | 307 | 3 | 45 |
| 3 | 307 | 3 | 40 |
| 4 | 328 | 5 | 36 |
| 5 | 331 | 4 | 36 |
| 6 | 331 | 4 | 35 |

Example 17

This example shows the results obtained in the sparged system using lanthanum-treated, acid-washed LZ-10 as catalyst.

Lanthanum-treated, acid-washed LZ-10 was prepared in the same way as in Example 10, but substituting lanthanum acetate for the calcium acetate, and tested in the sparged system described above. The results obtained are shown in Table XVII below.

Table XVII

| Time, hours | Temp. °C | Conversion | Selectivity |
|---|---|---|---|
| 1 | 285 | 10 | 61 |
| 4 | 350 | 23 | 74 |
| 7 | 425 | 49 | 83 |
| 11 | 325 | 36 | 81 |
| 14 | 325 | 23 | 75 |

Example 18

This example shows the results obtained in the sparged system using acid-washed LZ-10 as catalyst.

Acid-washed LZ-10 was prepared using the acid washing step but not the calcium treatment step of Example 10. The acid-washed LZ-10 thus prepared was tested in the sparged system described above, and the results obtained are shown in Table XVIII below.

Table XVIII

| Time, hours | Temp. °C | Conversion | Selectivity |
|---|---|---|---|
| 2 | 285 | 14 | 66 |
| 5 | 350 | 24 | 54 |
| 8 | 425 | 46 | 35 |
| 12 | 325 | 18 | 38 |
| 15 | 325 | 10 | 39 |

Example 19

M-8 zeolite was used as a catalyst in the pumped system described above. The results obtained are shown in Table XIX below.

Table XIX

| Time, hours | Temp. °C | Conversion | Selectivity |
|---|---|---|---|
| 0.7 | 319 | 17 | 64 |
| 1.5 | 319 | 16 | 65 |
| 2.0 | 319 | 28 | 61 |
| 3.5 | 365 | 32 | 64 |
| 4.2 | 365 | 25 | 69 |
| 5.2 | 364 | 22 | 69 |
| 6.5 | 393 | 37 | 63 |

Example 20

Zeolon 700 (a ferrierite sold commercially by Norton Chemical Process Products) was used as a catalyst in the pumped system described above. The results obtained are shown in Table XX below.

Table XX

| Time, hours | Temp. °C | Conversion | Selectivity |
|---|---|---|---|
| 0.7 | 336 | 14 | 84 |
| 1.5 | 339 | 13 | 82 |
| 2.2 | 338 | 6 | 76 |
| 3.2 | 377 | 23 | 77 |
| 4.5 | 378 | 29 | 79 |
| 5.2 | 377 | 26 | 78 |
| 6.0 | 377 | 23 | 77 |
| 6.7 | 377 | 21 | 76 |

**Claims**

1. A process for dehydrating an aldehyde to a diene, which process comprises contacting the aldehyde with a zeolite or a silica molecular sieve under conditions effective to dehydrate the aldehyde to the diene.

2. A process according to claim 1 wherein the aldehyde contains from about 4 to about 6 carbon atoms.

3. A process according to claim 2 wherein the aldehyde is 2-methylbutyraldehyde and the diene is isoprene.

4. A process according to claim 1 wherein the zeolite is an X or Y type zeolite, or a Y type zeolite which has been subjected to removal of aluminum from the lattice framework.

5. A process according to claim 4 wherein the Y type zeolite is selected from the group consisting of Y-52, Y-72 and Y-82 zeolites.

6. A process according to claim 5 wherein the Y type zeolite is a Y-82 zeolite.

7. A process according to claim 6 wherein the Y-82 zeolite is a phosphorus-treated Y-82 zeolite.

8. A process according to claim 4 wherein the Y type zeolite which has been subjected to removal of aluminum from the lattice framework is an LZ-10 LZ-20 or LZ-210 zeolite.

9. A process according to claim 8 wherein the LZ-zeolite is an LZ-10 or LZ-20 zeolite.

10. A process according to claim 8 wherein the zeolite has been washed prior to contact with the aldehyde.

11. A process according to claim 8 wherein, prior to contact with the aldehyde, the zeolite is treated to incorporate therein any one or more of an alkali metal, an alkaline earth metal, a rare earth metal, zinc, cadmium, manganese, aluminum, bismuth and phosphorus.

12. A process according to claim 9 wherein the zeolite has been acid washed prior to contact with the aldehyde.

13. A process according to claim 12, wherein, prior to contact with the aldehyde, the zeolite is treated to incorporate therein any one or more of an alkali metal, an alkaline earth metal, a rare earth metal, zinc, cadmium, manganese, aluminum, bismuth and phosphorus.

14. A process according to claim 13 wherein the zeolite is acid-washed, calcium-treated LZ-10, acid-washed, magnesium-treated LZ-10, acid-washed, barium-treated LZ-10, acid-washed, strontium-treated LZ-10, acid-washed, lanthanum-treated LZ-10, phosphorus-treated LZ-10 and acid-washed, magnesium-treated LZ-20.

15. A process according to claim 1 wherein the zeolite is a ferrierite.

16. A process according to claim 1 wherein the silica molecular sieve is S-115 or S-130.

17. A process according to claim 16 wherein the silica molecular sieve is S-115 or phosphorus-treated S-115.

18. A process according to claim 1 wherein the aldehyde is in the gaseous phase while being contacted with the zeolite or silica molecular sieve.

19. A process according to claim 18 wherein the aldehyde is mixed with an inert carrier gas while being contacted with the zeolite or silica molecular sieve.

20. A process according to claim 19 wherein the inert corner gas is nitrogen or carbon dioxide.

21. A process according to claim 19 wherein the inert carrier gas is steam.

22. A process according to claim 1 which is carried out at a weight hourly space velocity, based on the aldehyde, of from about 0.01 to about 40.

23. A process according to claim 22 which is carried out at a weight hourly space velocity, based on the aldehyde, of from about 1 to about 10.

24. A process according to claim 1 which is carried out at a temperature of about 150°C to about 500°C.

25. A process according to claim 24 which is carried out at a temperature of about 250°C to about 400°C.

26. A process according to claim 1 which is carried out at a pressure of from about 0.1 to about 200 atmospheres.

27. A process according to claim 26 which is carried out at a pressure of from about 0.1 to about 50 atmospheres.

28. A process according to claim 27 which is carried out at substantially atmospheric pressure.

29. A process according to claim 1 which is carried out at a conversion of at least about 10%.

30. A process according to claim 1 which is carried out at a selectivity to the diene of at least about 50%.

31. A process for the preparation of a diene, wherein an aldehyde of the formula

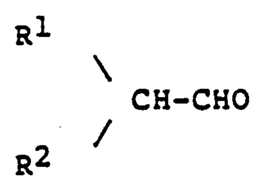

I

where $R^1$ and $R^2$ are identical or different, $R^1$ is alkyl with 1 to 3 carbon atoms or hydrogen and $R^2$ is alkyl with 1 to 8 carbon atoms or a hydrocarbon radical which together with $R^1$ forms a cyclohexyl radical, is converted at from 150°C to 600°C over a zeolite as a catalyst.

32. A process for the preparation of a diene of the formula

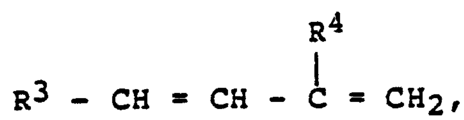

II

where $R^3$ is emthyl, ethyl or hydrogen and $R^4$ is methyl or ethyl, wherein 2-methylbutanal, 2-methylpentanal, 2-ethylhexanal, 3-methylbutanal, isovaleraldehyde or pivalaldehyde is passed at from 300° to 500°C over a zeolite as a catalyst.

33. A process as claimed in claim 31 wherein a zeolite of the pentasil type is used.

34. A process as claimed in claim 31, wherein an aluminosilicate zeolite is used.

35. A process as claimed in claim 31, wherein a borosilicate zeolite is used.

36. A process as claimed in claim 31, wherein an iron silicate zeolite is used.